# EUROPEAN PATENT SPECIFICATION

(11) **EP 4 452 158 B1**
(45) Date of publication and mention of the grant of the patent: **06.05.2026**
(21) Application number: 23813079.3
(22) Date of filing: 21.11.2023
(51) Int. Cl.: A61F 13/05

(54) **SURGICAL MESH SECURING DEVICE FOR WOUND CLOSURE SYSTEM**
CHIRURGISCHE NETZSICHERUNGSVORRICHTUNG FÜR WUNDVERSCHLUSSSYSTEM
DISPOSITIF DE FIXATION DE FILET CHIRURGICAL POUR SYSTÈME DE FERMETURE DE PLAIE

(30) Priority: 22.11.2022 US 202217991945
(43) Date of publication of application: 30.10.2024
(73) Proprietor: Cilag GmbH International, 6300 Zug (CH)
(72) Inventor: QUINTERO, Julian, Raritan, New Jersey 08869 (US); KRIKSUNOV, Leo, Raritan, New Jersey 08869 (US)
(74) Representative: Carpmaels & Ransford LLP
(86) International application number: PCT/IB2023/061724
(87) International publication number: WO 2024/110858

(56) References cited:
- US-A1- 2007 055 205
- US-A1- 2010 042 058
- US-A1- 2013 102 945
- US-A1- 2013 231 619

## Description

### BACKGROUND

A wound closure system (also referred to as a skin closure system) may be used at the conclusion of a surgical procedure on a patient to close a wound (e.g., a surgical incision) that was formed in the patient's skin for accessing a target anatomical structure. By way of example, wound closure systems may include components such as sutures, substrates, and/or liquid topical skin adhesives that are applied by a surgeon to approximate the edges of the wound and, in some cases, form a stable and protective layer over the wound that promotes efficient healing. In some instances, one or more components of the applied wound closure system may be absorbed by the patient during the healing process. Following healing of the wound, remaining components of the wound closure system may be removed from the skin by a surgeon, and/or they may automatically separate from the skin such that they may be discarded by the patient.

US2007055205 describes a protective device having a substantially rigid casing for placement on a patient's skin, wherein the casing has a hollow interior facing the patient's skin and a lip.

US2013102945 describes a light-weight, semi-rigid wound shield that is said to protect wounds and intradermal therapy ports from impacts between therapy sessions.

US2010042058 describes a drainage system that is said to extract fistula-produced discharge, or other bodily fluids, from an injury, such as a burn, laceration, or contusion.

US2013231619 describes a wound management device comprising at least one wall element of flexible sheet material, the device being securable to skin around a wound site to define a space wherein the wound is accessible; and a connector element held to the at least one wall element and affording at least one passage for external communication with the space; wherein the connector element includes at least one formation through which the or a passage extends, for engagement by a member for fluid supply to or removal from the space.

While various wound closure systems and associated components and methods have been made and used, it is believed that no one prior to the inventors has made or used the invention described in the appended claims.

### BRIEF DESCRIPTION OF THE DRAWINGS

The accompanying drawings, which are incorporated in and constitute a part of this specification, illustrate embodiments of the invention, and, together with the description given below, serve to explain the principles of the present invention.
FIG. 1 depicts a perspective view of an example of a wound closure system that includes a wound closure device, an adhesive applicator, and an adhesive spreader;
FIG. 2 depicts a disassembled perspective view of the wound closure device of FIG. 1, showing a mesh layer, a pressure sensitive adhesive layer, and a removable backing layer;
FIG. 3A depicts a perspective view of the wound closure device of FIG. 1 aligned longitudinally with a wound in the skin of a patient, showing a central section of the backing layer having been removed;
FIG. 3B depicts a perspective view of the wound closure device of FIG. 1 applied to the patient's skin over the wound to approximate the edges of the wound;
FIG. 3C depicts a perspective view of the wound closure device of FIG. 1 applied to the patient's skin over the wound, showing remaining sections of the backing layer having been removed so the wound closure device is fully adhered to the skin;
FIG. 3D depicts a perspective view of the wound closure device of FIG. 1 applied to the patient's skin over the wound, showing application of a liquid topical skin adhesive onto the mesh layer with the adhesive applicator;
FIG. 3E depicts a perspective view of the wound closure device of FIG. 1 applied to the patient's skin over the wound, showing the adhesive spreader positioned against the patient at the start of an adhesive spreading stroke for spreading the applied topical skin adhesive over and through the wound closure device;
FIG. 3F depicts a perspective view of the wound closure device of FIG. 1 applied to the patient's skin over the wound, showing the adhesive spreader during a subsequent portion of the adhesive spreading stroke;
FIG. 4 depicts a perspective view of another illustrative wound closure device;
FIG. 5 depicts a perspective view of a cannula that may be used in conjunction with the wound closure device of FIG. 4;
FIG. 6A depicts a perspective view of the wound closure device of FIG. 4 aligned longitudinally with a wound in the skin of a patient;
FIG. 6B depicts a perspective view of the wound closure device of FIG. 4 applied to the patient's skin over the wound to approximate the edges of the wound;
FIG. 6C depicts a perspective view of the wound closure device of FIG. 4 applied to the patient's skin over the wound, where the cannula of FIG. 5 is inserted within a cannula housing of the wound closure device such that the cannula is aligned longitudinally with the wound in the skin of the patient;
FIG. 6D depicts a perspective view of the wound closure device of FIG. 4 applied to the patient's skin over the wound, and the cannula of FIG. 5 housed within the cannula housing of FIG. 6C, showing application of a liquid topical skin adhesive onto the mesh layer with the adhesive applicator;
FIG. 6E depicts a perspective view of the wound closure device of FIG. 4 applied to the patient's skin over the wound, and the cannula of FIG. 5 housed within the cannula housing of FIG. 6C, showing spreading of the topical skin adhesive over and through the wound closure device with the adhesive spreader;
FIG. 7A depicts a cross-sectional view, taken along line 7-7 of FIG. 6E, of the wound closure device of FIG. 4 and the cannula of FIG. 5 applied to the patient skin with topical skin adhesive covering the wound closure device;
FIG. 7B depicts a cross-sectional view, taken along line 7-7 of FIG. 6E, of the wound closure device of FIG. 4 and the cannula of FIG. 5 applied to the patient skin with topical skin adhesive covering the wound closure device, with secreted fluids accumulated within the wound;
FIG. 7C depicts a cross-sectional view, taken along line 7-7 of FIG. 6E, of the wound closure device of FIG. 4 and the cannula of FIG. 5 applied to the patient skin with topical skin adhesive covering the wound closure device, with secreted fluids being suctioned from the wound via the cannula;
FIG. 8A depicts a cross-sectional view of a base textile mesh of another illustrative wound closure device applied to the patient skin;
FIG. 8B depicts a cross-sectional view of the base textile mesh of FIG. 8A aligned with a top textile mesh and a cannula of the wound closure device of FIG. 8A;
FIG. 8C depicts a cross-sectional view of the base textile mesh of FIG. 8A attached to the top textile mesh of FIG. 8B, thereby forming a cannula housing to contain the cannula of FIG. 8B, with topical skin adhesive applied to the wound closure device;
FIG. 9 depicts a perspective view of an alternative wound closure device that may be used in conjunction with the cannula of FIG. 5;
FIG. 10 depicts a perspective view of an alternative wound closure device that may be used in conjunction with the cannula of FIG. 5;
FIG. 11 depicts a perspective view of an alternative wound closure device that may be used in conjunction with the cannula of FIG. 5;
FIG. 12 depicts a perspective view of an alternative wound closure device that may be used in conjunction with the cannula of FIG. 5;
FIG. 13 depicts a longitudinal cross-sectional view of the wound closure device of FIG. 4 coupled with an alternative cannula;
FIG. 14 depicts a longitudinal cross-sectional view of an alternative cannula;
FIG. 15 depicts a longitudinal cross-sectional view of an alternative cannula;
FIG. 16 depicts a longitudinal cross-sectional view of an alternative cannula;
FIG. 17 depicts a longitudinal cross-sectional view of an alternative cannula; and
FIG. 18 depicts a longitudinal cross-sectional view of an alternative cannula.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

The invention is defined by the appended claims.

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

For clarity of disclosure, the terms "proximal" and "distal" are defined herein relative to a human or robotic operator of the surgical instrument. The term "proximal" refers the position of an element closer to the human or robotic operator of the surgical instrument and further away from the surgical end effector of the surgical instrument. The term "distal" refers to the position of an element closer to the surgical end effector of the surgical instrument and further away from the human or robotic operator of the surgical instrument. It will be further appreciated that, for convenience and clarity, spatial terms such as "side," "upwardly," and "downwardly" also are used herein for reference to relative positions and directions. Such terms are used below with reference to views as illustrated for clarity and are not intended to limit the invention described herein.

Furthermore, the terms "about," "approximately," and the like as used herein in connection with any numerical values or ranges of values are intended to encompass the exact value(s) referenced as well as a suitable tolerance that enables the referenced feature or combination of features to function for the intended purpose described herein.

### I. Wound Closure System

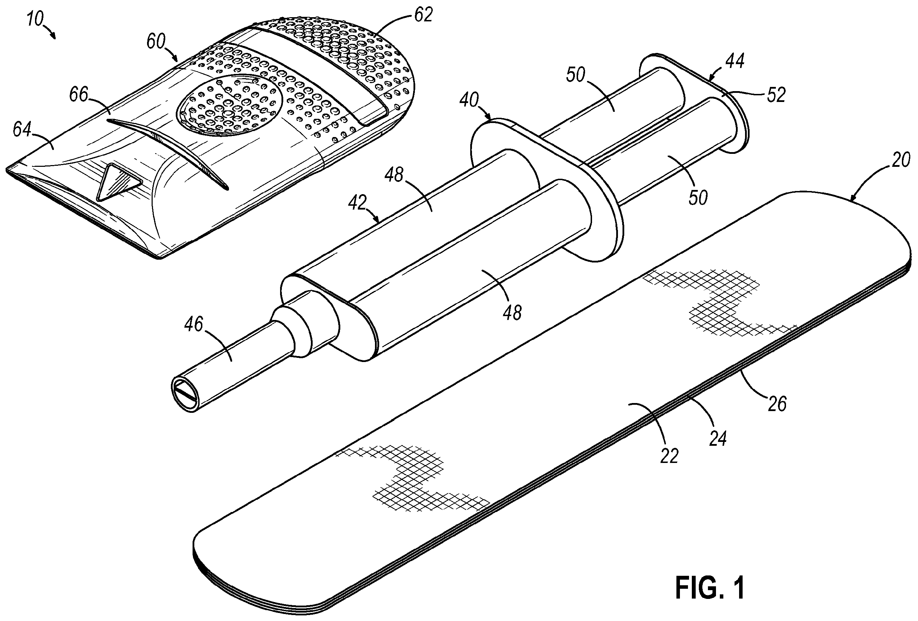

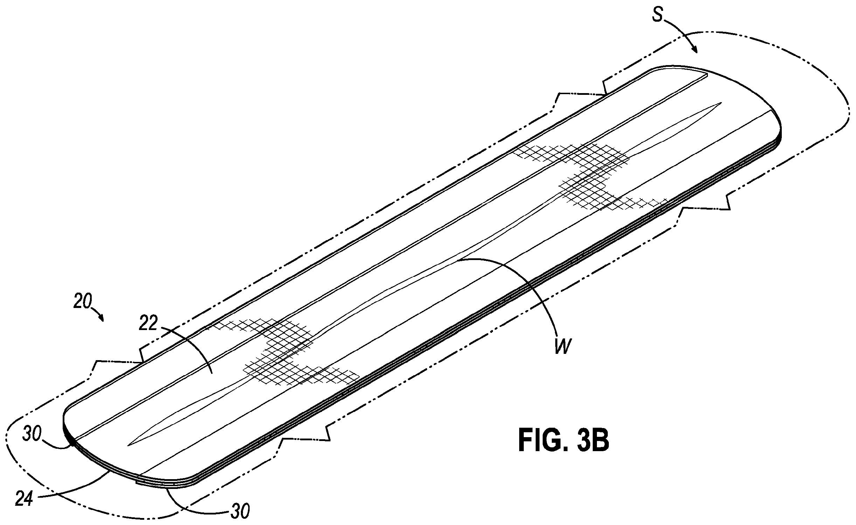

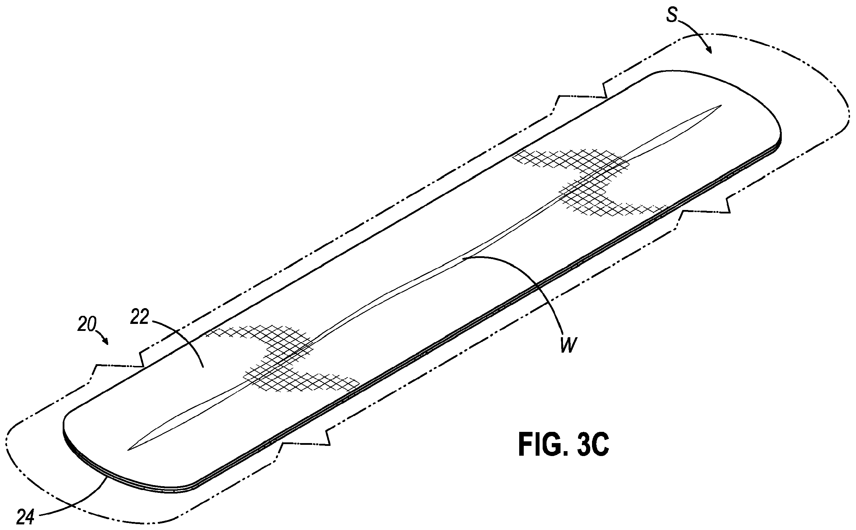

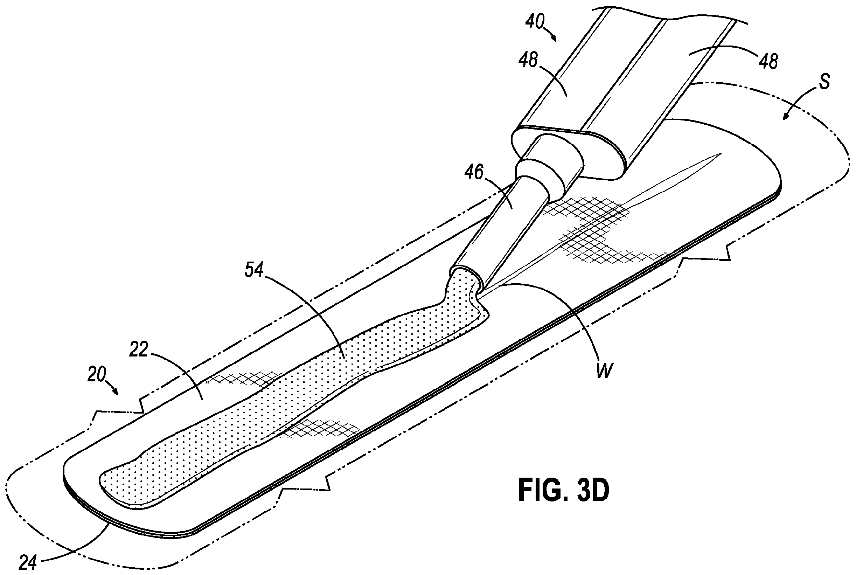

FIG. 1 shows an example of a wound closure system (10) that includes a wound closure device (20) (also referred to as a patch) configured to be applied to a patient's wound (W), an adhesive applicator (40) configured to apply a topical skin adhesive (54) (see FIG. 3D) to the applied wound closure device (20), and an adhesive spreader (60) configured to spread the applied topical skin adhesive (54) onto and through wound closure device (20). Each of these components is described in greater detail below.

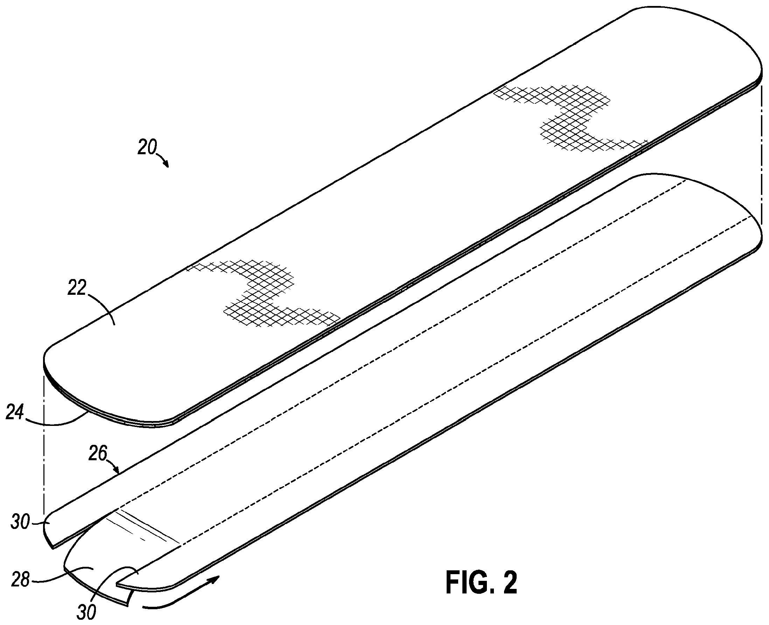

As shown best in FIG. 2, wound closure device (20) of the present version has an elongate, generally rectangular shape and a triple layer construction. More specifically, wound closure device (20) includes a layer of substrate in the form of a textile mesh (22), a layer of pressure sensitive adhesive (24) formed as a continuous or non-continuous coating along the lower skin-facing side of mesh (22), and a layer of backing (26) removably applied to the lower side of pressure sensitive adhesive (24). It will be understood that the term "wound closure device" as used herein encompasses wound closure device (20) both with and without backing (26), which may be removed and discarded during application of wound closure device (20) to a patient, as described in greater detail below.

Mesh (22) is configured to retain a liquid topical skin adhesive and may be formed of polyethylene (PET) or any other suitable surgical textile material. Pressure sensitive adhesive (24) is configured to enable wound closure device (20) to self-adhere to a patient's skin in response to a pressure being applied to the upper side of mesh (22) during its application by a surgeon. Backing (26) serves to protect pressure sensitive adhesive (24) before application of wound closure device (20) to the patient. In the present version, backing (26) includes elongate arrays of perforations that extend longitudinally and define an elongate central backing section (28) and a pair of elongate side backing sections (30). Though each backing section (28, 30) is shown as generally rectangular in the present version, backing sections (28, 30) may be of various alternative shapes, sizes, and quantities in other versions.

As shown in FIG. 1, adhesive applicator (40) includes an applicator body (42), a plunger unit (44) slidably received within an open proximal end of applicator body (42), and a static mixer (46) secured to a distal end of applicator body (42). Applicator body (42) includes a pair of barrels (48) arranged side by side, where each barrel (48) houses a respective part of a two-part liquid topical skin adhesive. Plunger unit (44) includes a pair of plungers (50) that are arranged side by side and are interconnected at their proximal ends by a bridge (52). Each plunger (50) is actuatable distally through a respective barrel (48) of applicator body (42) to force the corresponding liquid adhesive part distally into static mixer (46). Static mixer (46) is configured to receive the first and second adhesive parts and direct them around and through a series of static baffles and passages (not shown) that mix the two adhesive parts together into a homogenous liquid adhesive (54) that is then dispensed through an open distal end of static mixer (46), as shown in FIG. 3D described below.

In the present version, liquid topical skin adhesive (54) is in the form of a silicone-based topical skin adhesive that is configured to cure on skin at body temperature in less than two minutes. Once cured, topical skin adhesive (54) remains elastomeric such that a given section of cured adhesive (54) is configured to stretch up to 160% of its cured length and then fully recover to the cured length. Accordingly, wound closure system (10) may be particularly effective for use on actuatable body parts of a patient such as a knee, wrist, elbow, or other joint, for example.

As also shown in FIG. 1, adhesive spreader (60) of the present version has a monolithic body that includes a proximal body portion (62) configured to be gripped by a user, a distal body portion (64) that terminates at a tip configured to spread applied topical skin adhesive (54), and an intermediate flexural body portion (66) that interconnects the proximal and distal body portions (62, 64). Flexural body portion (66) is configured to elastically deform so that distal body portion (64) angularly deflects relative to proximal body portion (62) to promote effective spreading of topical skin adhesive (54) along wound closure device (20) with a suitable normal force within a predetermined range.

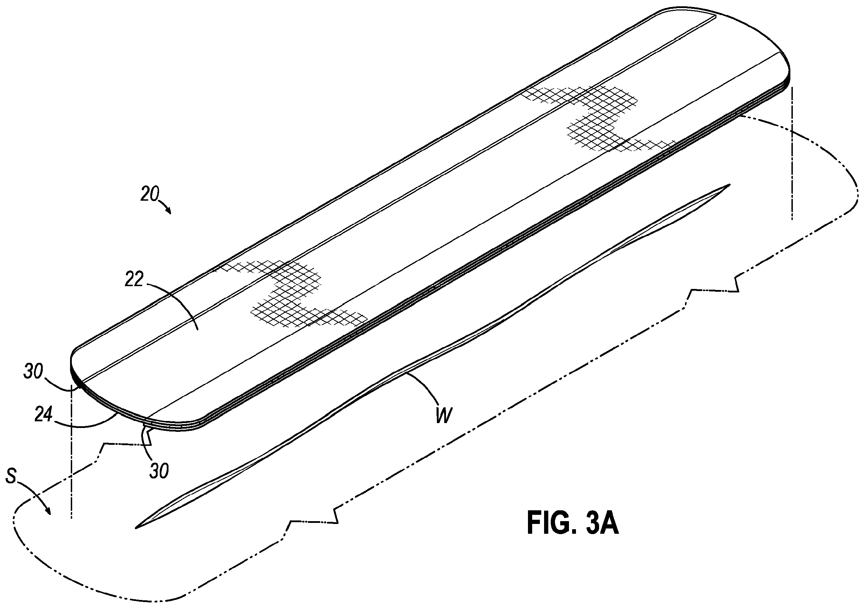

FIGS. 3A-3D show an example of wound closure system (10) being used to close a wound (W) formed in the skin (S) of a patient. In some procedures, wound (W) may be at least partially closed with one or more sutures, for example at deeper portions of wound (W), prior to use of wound closure system (10). Additionally, before wound closure device (20) is applied to the patient, it may be trimmed by a surgeon to any suitable shape as desired.

As shown in FIG. 3A, central backing section (28) is removed from wound closure device (20) to expose a central window of mesh (22) and pressure sensitive adhesive (24), and an imaginary centerline of wound closure device (20) is aligned longitudinally with the edges of wound (W). Wound closure device (20) is then applied to the patient skin (S) over wound (W) and the surgeon applies pressure to the central portion of mesh (22) to force pressure sensitive adhesive (24) to adhere to the skin (S), thus fixing the edges of wound (W) relative to one another. Before this step, the edges of wound (W) may be held in an approximated state by the surgeon. Alternatively, during this step the central portion of wound closure device (20) may be applied in a laterally alternating manner to approximate the edges of wound (W) during application. With either approach, wound closure device (20) is configured to hold the edges of wound (W) in an approximated state following this initial step of application. Once the surgeon is satisfied with the position of wound closure device (20) relative to wound (W), the surgeon may then remove the remaining two side backing sections (30) to adhere the reminder of wound closure device (20) to skin (S).

As shown in FIG. 3D, adhesive applicator (40) is then used to apply a pattern of topical skin adhesive (54) to the upper side of mesh (22) of the applied wound closure device (20). While adhesive applicator (40) is shown applying a linear bead of topical skin adhesive (54) in the present version, it will be appreciated that various other patterns of topical skin adhesive (54) may be applied in other versions, such as a T-shaped pattern or a wave-shaped pattern as disclosed in US2024165292A1, entitled "Application of Topical Skin Adhesive to Surgical Mesh," filed on even date herewith.

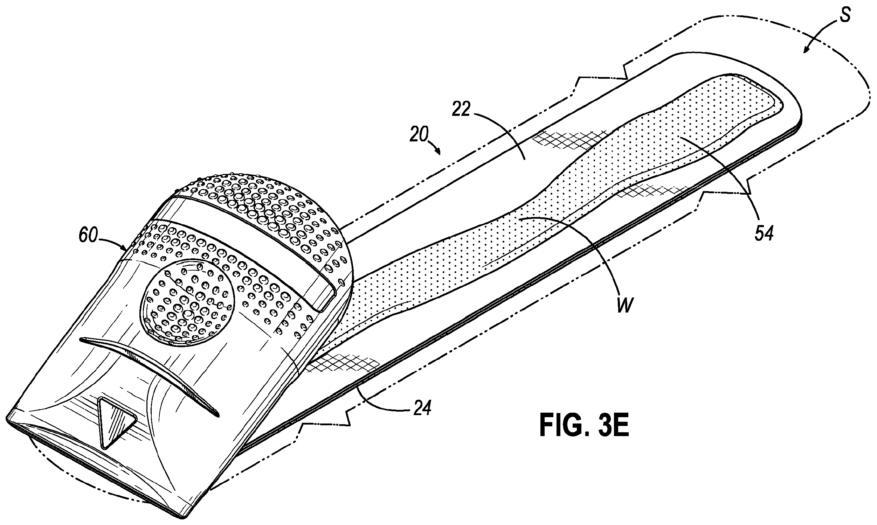

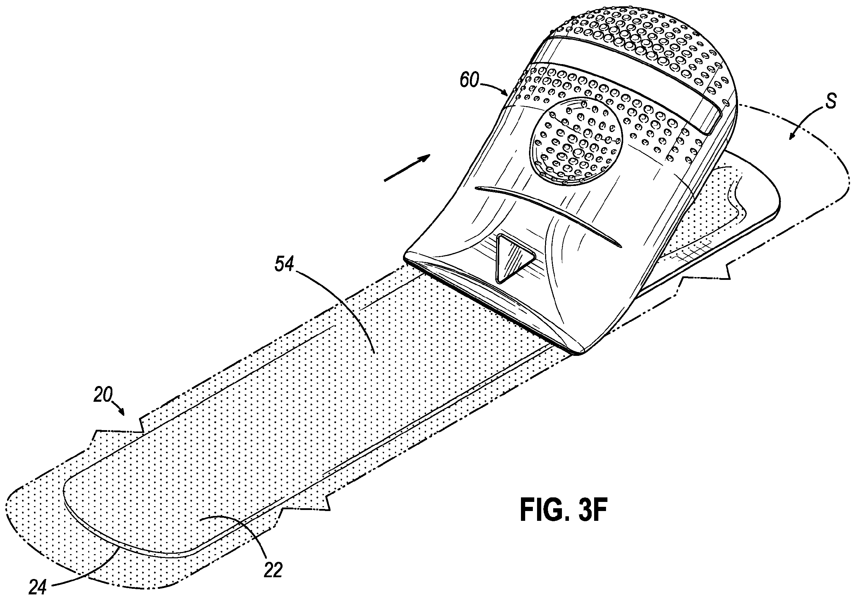

As shown in FIGS. 3E-3F, adhesive spreader (60) is then used to spread the applied topical skin adhesive (54) uniformly over wound closure device (20) to force the topical skin adhesive (54) through the layers of mesh (22) and pressure sensitive adhesive (24) and directly against wound (W) and the surrounding skin (S). In that regard, the layers of mesh (22) and pressure sensitive adhesive (24) may be at least partially permeable to permit forced passage of topical skin adhesive (54) therethrough. As shown in FIG. 3E, flexural body portion (66) of adhesive spreader (60) may be in a non-deformed state when adhesive spreader (60) is first positioned against wound closure device (20) at the beginning of an adhesive spreading stroke. As adhesive spreader (60) is dragged longitudinally along wound closure device (20), the input force exerted by the user may cause flexural body portion (66) to elastically deform such that distal body portion (64) angularly deflects relative to proximal body portion (62), as shown in FIG. 3F. The degree of deformation of flexural body portion (66) may be directly related to the viscosity of topical skin adhesive (54). In particular, a greater viscosity may require that the user exert a greater input force through proximal body portion (62) to effectively spread topical skin adhesive (54) over and through wound closure device (20), such that the flexural body portion (66) deforms a relatively greater amount. Conversely, a lesser viscosity may require a lesser input force such that the flexural body portion (66) deforms less or not at all.

Optionally, topical skin adhesive (54) may also be spread over adjacent portions of skin (S) not covered by wound closure device (20) to ensure that an entirety of mesh (22) is embedded with topical skin adhesive (54). For instance, and by way of example only, topical skin adhesive (54) may be spread onto at least 1 cm of skin (S) about the entire outer perimeter of the applied wound closure device (20). Once topical skin adhesive (54) has been fully spread over wound closure device (20), any topical skin adhesive (54) on skin (S) beyond the perimeter of device (20) may then be wiped away with sterile gauze, for example. Additionally, in some instances, a quantity of topical skin adhesive (54) may be applied between the edges of wound (W) before wound closure device (20) is applied to the skin (S). The applied topical skin adhesive (54) then cures within and over wound closure device (20) to form a composite microbial barrier over wound (W) that maintains a protective environment that promotes efficient healing. Following healing of wound (W), wound closure device (20) may be removed from the skin (S) manually (e.g., by a surgeon) or it may automatically separate from the skin (S) such that it may be discarded by the patient.

Wound closure system (10) may be further configured and operable in accordance with at least some of the teachings of U.S. Pat. Pub. No. 2021/0369258, entitled "Systems, Devices and Methods for Dispensing and Curing Silicone Based Topical Skin Adhesives," published December 2, 2021; U.S. Pat. Pub. No. 2021/0371190, entitled "Systems, Methods and Devices for Aerosol Spraying of Silicone Based Topical Skin Adhesives for Sealing Wounds," published December 2, 2021; U.S. Pat. Pub. No. 2021/0371658, entitled "Novel Topical Skin Closure Compositions and Systems," published December 2, 2021; U.S. Pat. Pub. No. 20210369639, entitled "Novel Antimicrobial Topical Skin Closure Compositions and Systems," published December 2, 2021; U.S. Pat. Pub. No. 2021/0369276, entitled "Anisotropic Wound Closure Systems," published December 2, 2021; and/or U.S. Pat. App. No. 17/667,950, entitled "Gas Sterilizable Syringes Having Apertures Covered By Gas Permeable Barriers For Enabling Ingress and Egress of Sterilization Gases While Preventing Leakage of Flowable Materials," filed on February 9, 2022.

### II. Illustrative Wound Closure Device with Cannula Housing

As mentioned above, after wound closure device (20) is applied to wound (W) in accordance with the description above, topical skin adhesive (54) is suitably applied and spread within and over wound closure device (20) to form a composite microbial barrier over wound (W), thereby maintaining a protective environment that promotes effective healing. Such a microbial barrier may effectively seal the wound (W) from the external environment.

In some instances, after wound closure device (20) and topical adhesive (54) are suitably applied over wound (W), exudate and/or other matter may accumulate within wound (W). With wound (W) effectively sealed from the external environment, accumulation of exudate and/or other matter within wound (W) may lead to undesirable consequences, such as infection, inhibiting wound closure and/or other undesirable consequences that would be apparent to one skilled in the art in view of the teachings herein. Therefore, it may be desirable to have a wound closure device (20) capable of removing accumulated exudate and/or other matter after wound closure device (20) and topical adhesive (54) are suitably applied.

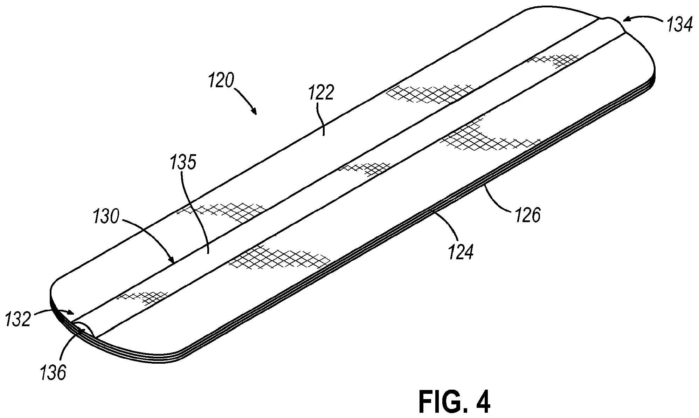

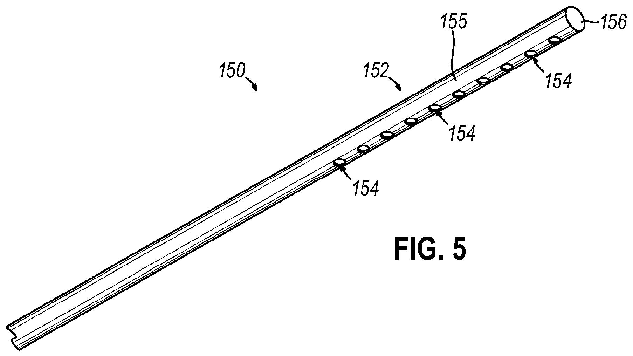

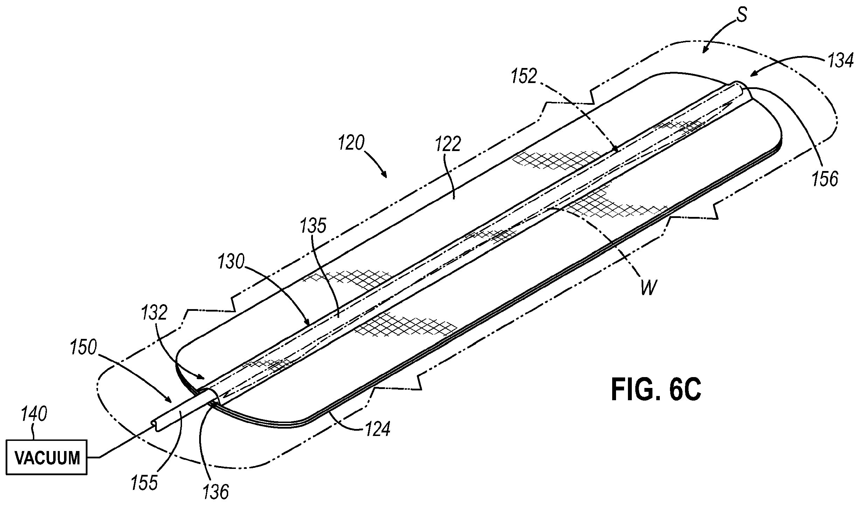

FIG. 4 shows an illustrative wound closure device (120), while FIG. 5 shows an illustrative cannula (150) that may be used in conjunction with wound closure device (120). As will be described in greater detail below, cannula (150) may be inserted into a cannula housing (130) of wound closure device (120) such that cannula (150) may provide fluid communication between an effectively sealed wound (W) and a suitable fluid and/or suction source. In the current example, wound closure device (120) and cannula (150) may be used together in conjunction with topical adhesive (54) and a vacuum source (140) *(see* FIGS. 6C-6E) in order to provide suitable negative pressure within wound (W) to remove accumulated exudate and/or other matter, while providing a suitable microbial barrier in accordance with the description herein.

Wound closure device (120) may be substantially similar to wound closure device (20) described above. Therefore, wound closure device (120) includes a textile mesh (122), a pressure sensitive adhesive (124), and backing (126); which may be substantially similar to textile mesh (22), pressure sensitive adhesive (24), and backing (26) described above, respectively, with differences elaborated below. Therefore, mesh (122) is configured to absorb liquid topical skin adhesive (54); backing (126) may be selectively removed to reveal pressure sensitive adhesive (124); and sensitive adhesive (124) is configured to enable wound closure device (120) to self-adhere to a patient's skin in response to a pressure being applied to the upper side of mesh (122) during its application by a surgeon.

Additionally, textile mesh (122) also includes cannula housing (130) which longitudinally extends from an open proximal end (130) toward a distal end (134). Cannula housing (130) is located on wound closure device (120) such that once wound closure device (120) is suitably attached on skin (S) of patient, cannula housing (130) is suitably adjacent relative to wound (W). In some instances, cannula housing (130) is suitably adjacent with wound (W) to thereby extend at least partially along the length of wound (W).

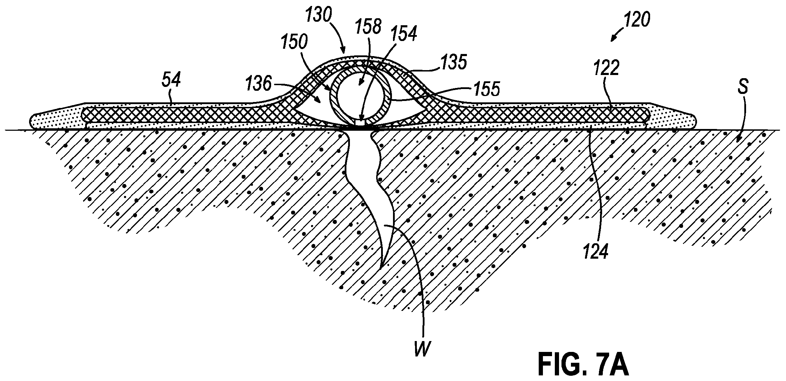

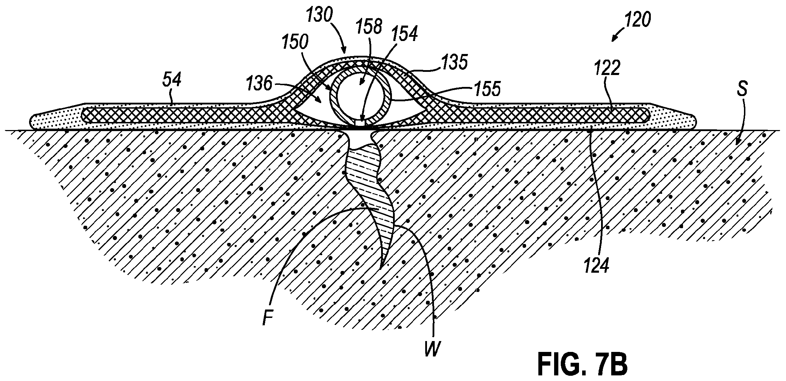

Cannula housing (130) includes a housing structure (135) defining a longitudinally extending channel (136) with an access opening at open proximal end (132). Cannula housing (130) is dimensioned to receive a portion of cannula (150) shown in FIG. 5. In particular, a distal tip (156) of cannula (150) may inserted into longitudinally extending channel (136) via open proximal end (132) until array of apertures (154) are suitably contained within cannula housing (130). As will be described in greater detail below, array of apertures (154) may be oriented within cannula housing (130) to provide fluid communication between wound (W) and a lumen (158) *(see* FIGS. 7A-7C) defined by cannula (150). Therefore, textile mesh (122), housing structure (135) and/or suitable portions of wound closure device (120) are configured to provide fluid communication between channel (136) and the underside of wound closure device such that array of apertures (154) are in fluid communication with wound (W) after suitable application of wound closure device (120), cannula (150), and topical adhesive (54) in accordance with the description herein

Longitudinally extending channel (136) may extend from open proximal end (130) toward distal end (134). In some instances, longitudinally extending channel (136) terminates at distal end (134). In other instances, longitudinally extending channel (136) extends though distal end (134) such that distal end (134) is also open. In other instances, longitudinally extending channel terminates prior to distal end (134). Therefore, longitudinally extending channel (136) may have any suitable length respective to the rest of wound closure device (120) as would be apparent to one skilled in the art in view of the teachings herein.

In the current aspect of the disclosure, housing structure (135) is formed of the same material as textile mesh (122) and extends above a top surface of the rest of textile mesh (122). In some aspects of the disclosure, housing structure (135) may be formed of a suitable different material as compared to textile mesh (122) as would be apparent to one skilled in the art in view of the teachings herein. Housing structure (135) may be formed of any suitable material and be located at any suitable location relative to textile mesh (122) as should be apparent to one skilled in the art in view of the teachings herein.

Turning to FIG. 5, cannula (150) includes a cannula body (155) that extends into a distal end (152) that terminates into distal tip (156). Distal tip (156), as shown in the current example, is closed off. In some aspects of the disclosure, distal tip (156) may be open. Distal end (152) of cannula body (155) is dimensioned to be inserted into channel (136) of cannula housing (130). After insertion in cannula housing (130), distal end (152) of cannula body (154) may be configured to remain substantially fixed relative to cannula housing (130) such that cannula (150) is not accidentally removed from the interior of housing structure (135). As will be described in greater detail below, cannula (150) and/or the interior of cannula housing (130) may have additional suitable features in order to ensure cannula (150) remains suitable housed within channel (136).

Distal end (152) of cannula body (155) also defines array of apertures (154) providing fluid communication between as exterior of cannula body (155) and lumen (158) *(see* FIGS. 7A-7C) defined by cannula body (155). In the current aspect of the disclosure, apertures (154) are aligned in a longitudinal array on cannula body (155). Distal end (152) of cannula body (155) may be oriented within cannula housing (130) such that apertures (154) are facing toward wound (W) once suitably inserted into cannula housing (130) of wound closure device (120) *(see* FIGS. 7A-7C).

Cannula body (154) defines at least one lumen (158) *(see* FIGS. 7A-7C) in fluid communication with apertures (154). As best shown in FIGS. 6C-6E, cannula body (154) includes a suitable length such that a proximal end of cannula (150) may be operatively attached to a fluid and/or suction source, such as vacuum (140). A proximal end of cannula (150) may include any suitable structures in order to operatively couple with vacuum (140) or any other suitable fluid and/or suction source as would be apparent to those skilled in the art in view of the teachings herein.

Vacuum (140) may be configured to provide a suction source such that a suitable negative pressure may be generated within wound (W). Therefore, negative pressure generated within wound (W) may drive undesirable matter away from wound (W), though apertures (154), and through lumen (158) in order to remove any undesirable accumulated exudate and/or other matter as would be apparent to one skilled in the art.

In the current example, cannula (150) is in fluid communication with vacuum (140). In some aspects of the disclosure, cannula (150) may be configured to operatively attach to another fluid and/or suction source, such as a reservoir of suitable fluids, including but not limited to therapeutic agents, saline, etc. Additionally, while cannula (150) is shown with a single lumen (158) in fluid communication with aperture (154), cannula (150) may include multiple lumens that are fluidly isolated from each other and in fluid communication with their own sets of respective apertures (154). Therefore, cannula (150) may be configured to provide suction at wound (W), as well as deliver suitable fluids to wound (W). In some other instances, more than one cannula (150) may be utilized and inserted into cannula housing (130) or one or more cannula housings (130).

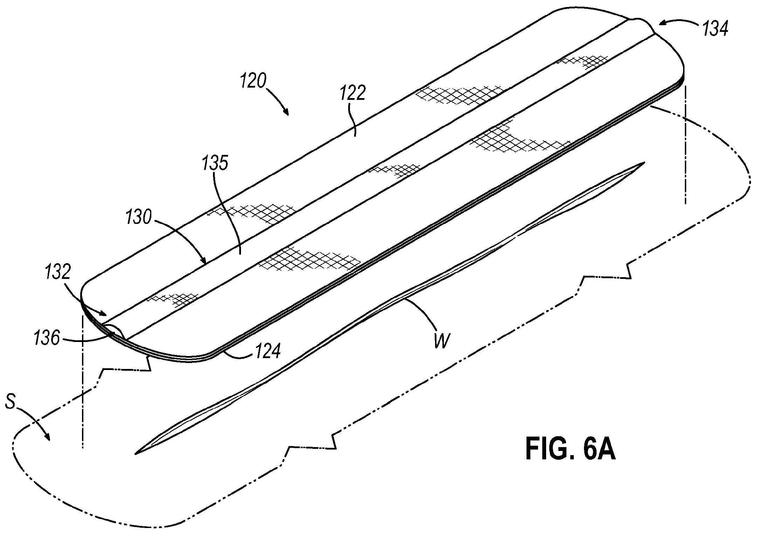

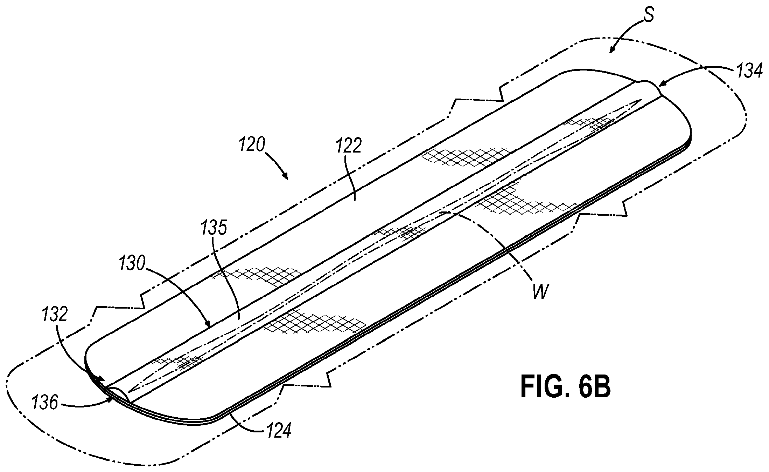

FIGS. 6A-6E show an example of wound closure system (120) being used in conjunction with cannula (150) to close a wound (W) formed in the skin (s) of a patient; while FIGS. 7A-7C show an illustrative use of would closure system (120), cannula (150), and vacuum (140) in order to generate negative pressure within wound (W) to remove accumulated exudate and/or other matter. As shown in FIG. 6A, backing (126) is suitably removed while mesh (122) and pressure sensitive adhesive (124) are suitably aligned with wound (W) such that cannula housing (130) is directly adjacent to wound (W). Next, as shown in FIG. 6B, wound closure device (120) is then applied to the patient skin (S) over wound (W) in similar fashion to wound closure device (20) described above. It should be understood that backing (126) of wound closure device (120) may be utilized in similar fashion to backing (26) of wound closure device described above. At the moment shown in FIG. 6B, pressure sensitive adhesive (124) suitably attaches wound closure device (120), at least temporarily, onto skin (S) of patient.

Next, as shown in FIG. 6C, distal end (152) of cannula (150) is inserted into longitudinally extending channel (136) via proximal opening (132). Distal end (152) may be inserted any suitable distance into channel (136) as would be apparent to one skilled in the art in view of the teachings herein. It should be understood that cannula (150) is oriented within channel (136) such that apertures (154) are facing toward wound (W) *(see* FIGS. 7A-7C). Distal end (152) may be suitably secured within channel (136) after insertion such that distal end (152) is not accidentally removed or otherwise inadvertently disassociate from cannula housing (130). Therefore, distal end (152) and/or the interior of cannula housing (130) may include features to help promote such association between the two.

As also shown in FIG. 6C, a proximal end of cannula (150) is suitably coupled to vacuum (140). Vacuum (140) may be located at any suitable location as would be apparent to one skilled in the art. Vacuum (140) is configured to selectively provide negative pressure within cannula (150). Vacuum (140) may include any suitable components as would be apparent to one skilled in the art in view of the teachings herein.

In some instances, cannula (150) may be a two-piece device, where distal end (152) terminates proximally past open proximal end (132) (thereby extending partially away from channel (136)), while the other piece of cannula (150) is longer and configured to selectively couple with distal end (152) in order to provide selective fluid communication with vacuum (140). In such instances, an open end of distal end (152) may include a removable cap or a seal in order to maintain a microbial seal when distal end (152) is not coupled to the second piece of cannula (150) and vacuum (140); while also allowing distal end (152) to selectively couple to vacuum (140).

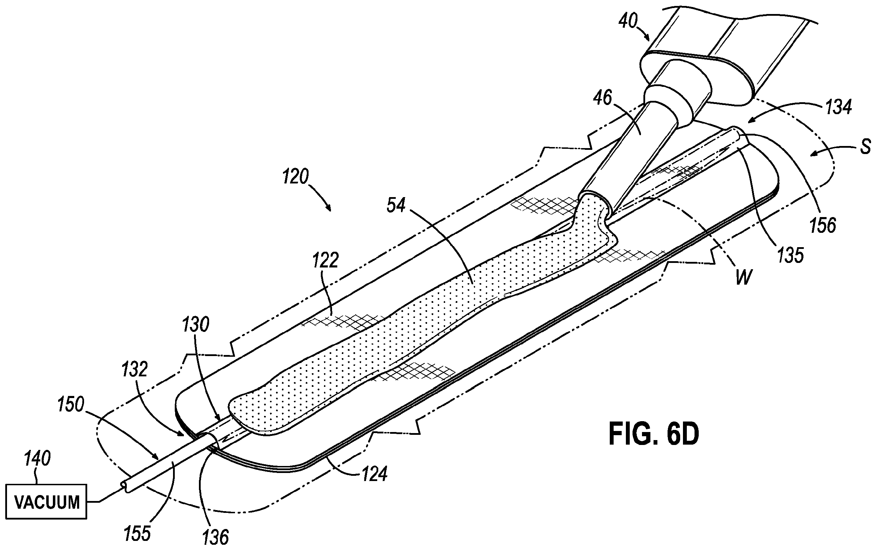

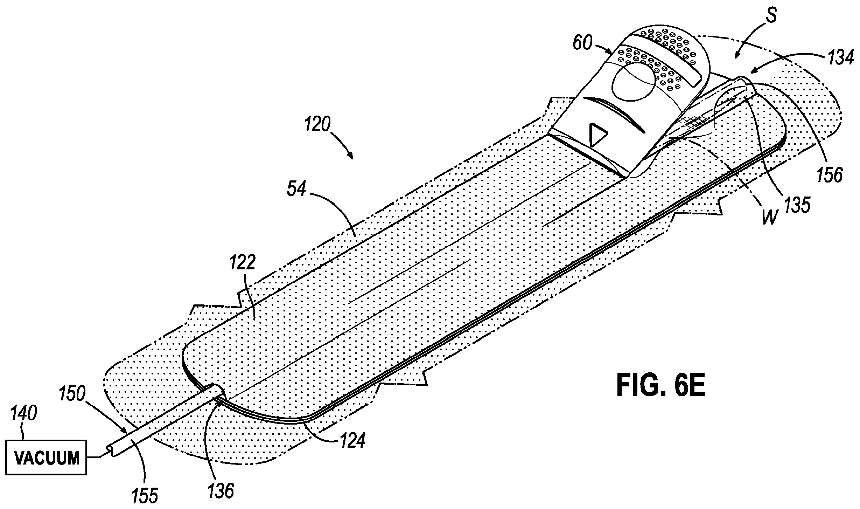

Next, as shown in FIG. 6D, adhesive applicator (40) is then used to apply a pattern of liquid topical skin adhesive (54) to the upper side of mesh (122) of the applied wound closure device (120). As shown in FIG. 6E, adhesive spreader (60) is then used to spread the applied topical adhesive (54) uniformly over wound closure device (120) as well as a portion of cannula (150) extending away from open proximal end (132) and surrounding skin (S) directly adjacent to such a portion of cannula (150). The applied topical adhesive (54) then cures within and over wound closure device (120), including proximal opening (132) and suitable portions of cannular (150), to form a composite microbial barrier over wound (W), thereby maintaining a protective environment that promotes efficient healing.

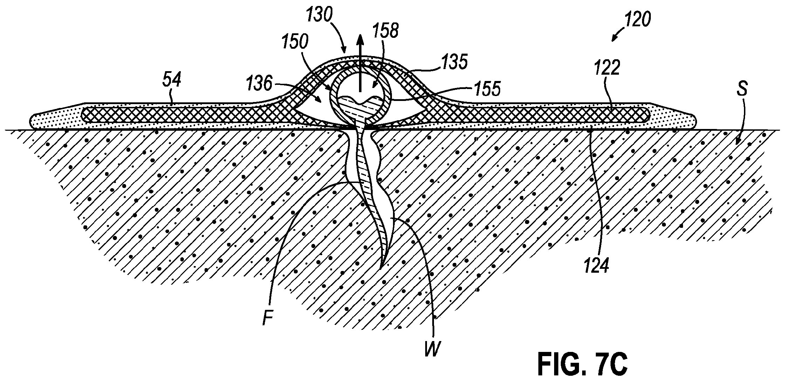

As shown between FIG. 7A-7B, after creating a microbial barrier over wound (W), exudate and/or other matter may tend to accumulate within wound (W) such that exudate may not be able to escape wound (W). In such instances, as shown in FIG. 7C, vacuum (140) may be selectively activated in order to generate a suitable negative pressure within wound (W). A suitable negative pressure may generate a suction force within wound (W) such that exudate (E) is suctioned away from wound (W), into lumen (158) via apertures (154), and toward vacuum (140). Allowing the removal of exudate (E) may promote faster healing of wound (W) or other benefits as would be apparent to one skilled in the art in view of the teachings herein. Negative pressure may be induced within wound (W) for any other suitable purposes as would be apparent to one skilled in the art in view of the teachings herein.

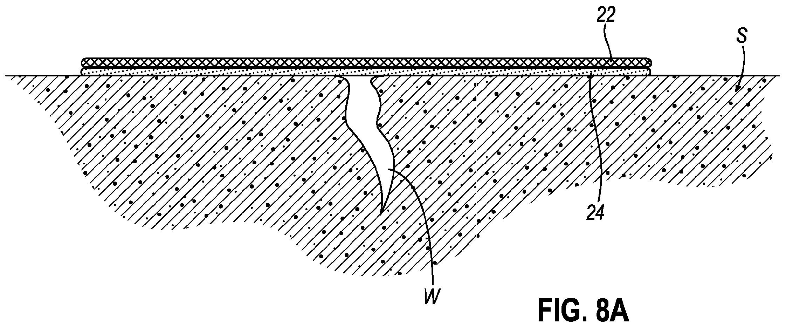

As mentioned above, cannula housing (130) formed from a single piece of textile mesh (122) such that cannula (150) may be inserted within channel (136) after wound closure device (120) is suitably attached to skin (S). However, in some instances, cannula (150) may be attached simultaneously with wound closure device (120). In other instances, cannula (150) may be interposed between two wound closure devices (20). FIGS. 8A-8C show another illustrative wound closure system (160) formed from two wound closure devices (20) and cannula (150) being interposed between each wound closure device (20) and being directly adjacent to wound (W).

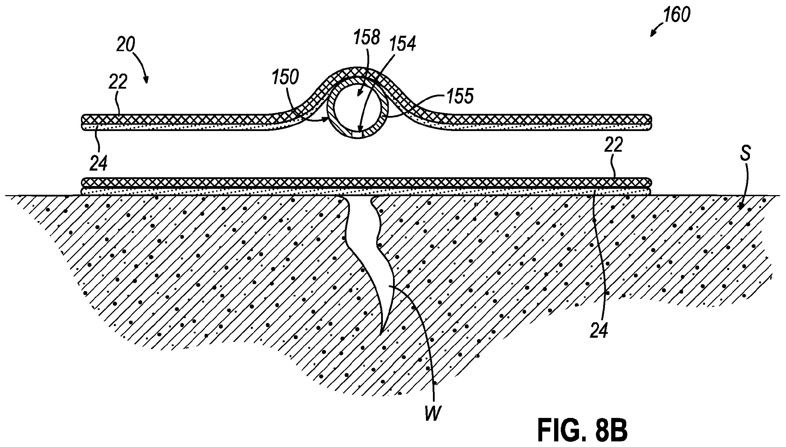

First, as shown in FIG. 8A, a first wound closure device (20) may be attached to skin (S), covering wound (W), in accordance with the description above. Next, as shown in FIG. 8B, a second wound closure device (20) may then be attached to the top of the first wound closure device (20) such that cannula (150) is interposed between each wound closure device (20) while apertures (154) are adjacent to wound (W). Pressure sensitive adhesive (24) of second wound closure device (20) may attach to textile mesh (22) of first wound closure device (20). Additionally, pressure sensitive adhesive (24) of second wound closure device (20) may attach to cannula (150), further promoting secure placement of cannula (150) relative to both wound closure devices (20).

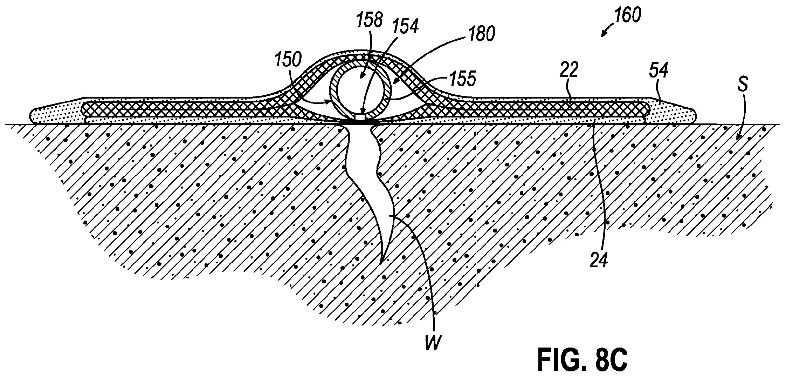

Once second wound closure device (20) is interposed on top of cannula (150) and first wound closure device (20), cannula (150) is housed within a longitudinally extending channel (180) defined by both wound closure devices (20) and cannula (150). Next, as shown in FIG. 8C, topical adhesive (54) may be applied in accordance with the description herein, thereby creating a suitable microbial seal while also providing fluid communication between cannula (150) and wound (W). With cannula (150) suitably placed adjacent to wound (W), cannula (150) may be attached to vacuum (140) such that suction may be provided at wound (W).

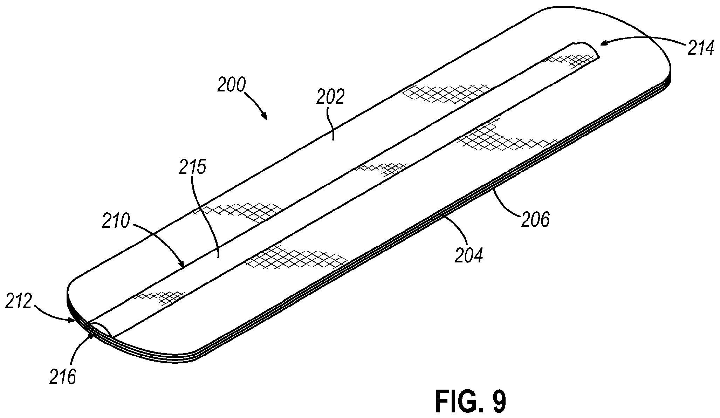

FIGS. 9-12 show alternative wound closure devices (200, 220, 240, 260) that may be readily incorporated in replacement of wound closure device (120) described above. Turning to FIG. 9, wound closure device (200) is substantially similar to wound closure device (120) described above, with differences elaborated below. Therefore, wound closure device (200) includes a textile mesh (202), a pressure sensitive adhesive (204), a backing (206), and a cannula housing (210); which are substantially similar to textile mesh (122), pressure sensitive adhesive (124), backing (126), and cannula housing (130) described above, with differences elaborated below.

In particular, cannula housing (210) includes a cannula housing structure (215) defining a longitudinally extending (216) channel. Cannula housing structure (215) extends from an open proximal end (212) to a distal end (214). Unlike cannula housing (130) describe above, distal end (214) in the current aspect of the disclosure terminates prior to the peripheral edge of textile mesh (202).

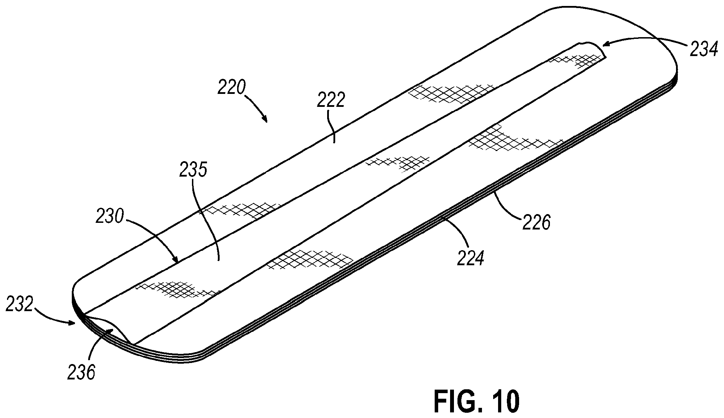

Turning to FIG. 10, wound closure device (220) is substantially similar to wound closure device (120) described above, with differences elaborated below. Therefore, wound closure device (220) includes a textile mesh (222), a pressure sensitive adhesive (224), a backing (226), and a cannula housing (230); which are substantially similar to textile mesh (122), pressure sensitive adhesive (124), backing (126), and cannula housing (130) described above, with differences elaborated below.

In particular, cannula housing (230) includes a housing structure (235) defining a longitudinally extending channel (236) and extending from an open proximal end (232) to a distal end (234). Unlike cannula housing (130) describe above, distal end (234) in the current aspect of the disclosure terminates prior to the peripheral edge of textile mesh (222). Additionally, housing structure (235) is tapered such that open proximal end (232) is wider compared to distal end (234). This may help promote easy insertion of cannula (150) into channel (236) of housing structure (235).

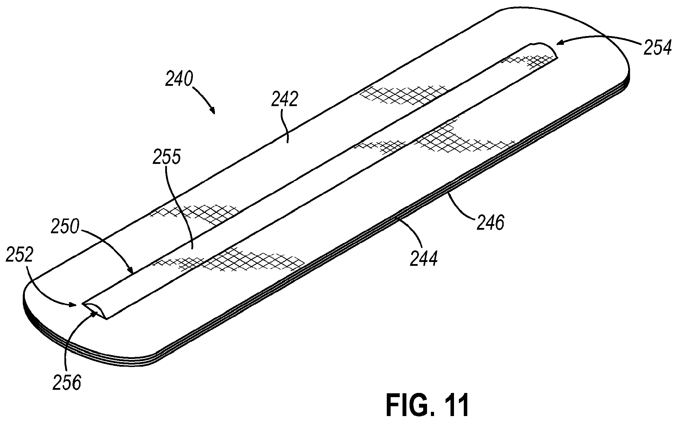

Turning to FIG. 11, wound closure device (240) is substantially similar to wound closure device (120) described above, with differences elaborated below. Therefore, wound closure device (240) includes a textile mesh (242), a pressure sensitive adhesive (244), a backing (246), and a cannula housing (250); which are substantially similar to textile mesh (122), pressure sensitive adhesive (124), backing (126), and cannula housing (130) described above, with differences elaborated below.

In particular, cannula housing (250) includes a housing structure (255) defining a longitudinally extending channel (256) and extending from an open proximal end (252) to a distal end (254). Unlike cannula housing (130) describe above, both proximal end (252) and distal end (254) in the current aspect of the disclosure terminate prior to the peripheral edge of textile mesh (242).

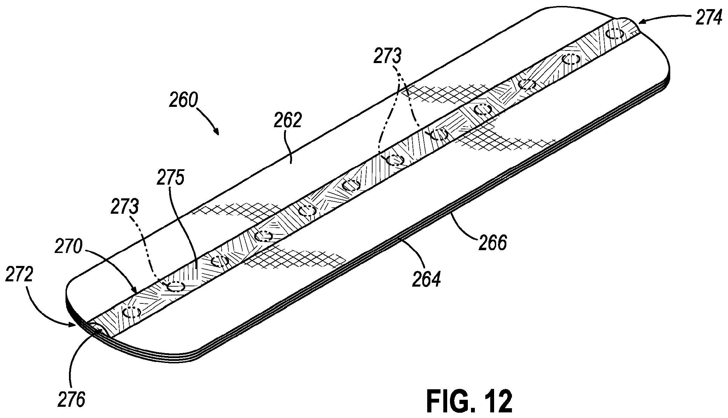

Turning to FIG. 12, wound closure device (260) is substantially similar to wound closure device (120) described above, with differences elaborated below. Therefore, wound closure device (260) includes a textile mesh (262), a pressure sensitive adhesive (264), a backing (266), and a cannula housing (270); which are substantially similar to textile mesh (122), pressure sensitive adhesive (124), backing (126), and cannula housing (130) described above, with differences elaborated below.

In particular, cannula housing (270) includes a housing structure (275) defining a longitudinally extending channel (276). Housing structure (275) extends from an open proximal end (272) to a distal end (274). Housing structure (275) is formed of a different material compared to textile mesh (262) and also defines apertures (273) on the bottom of cannula housing (270) to further promote fluid communication between channel (276) and wound (W) when wound closure device (260) is suitably attached to skin (S). Apertures (273) extend from the underside of wound closure device (260) and into channel (276).

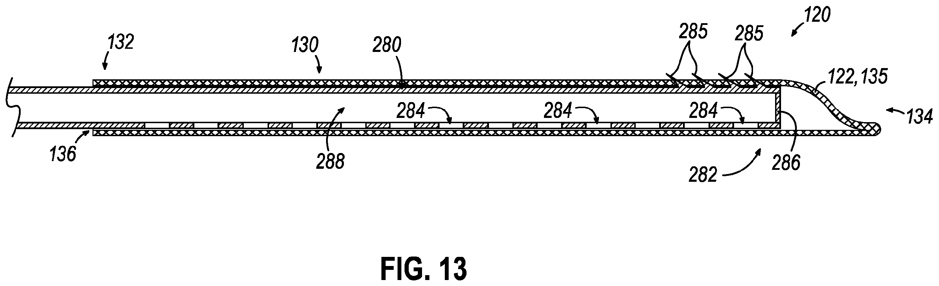

As mentioned above, cannula (150) may contain features to help secure an inserted cannula (150) relative to cannula housing (130). FIG. 13 shows an illustrative alternative cannula (280) that is substantially similar to cannula (150) described above, but with differences elaborated below. Cannula (280) includes a distal end (282), an array of apertures (284), a distal tip (286), and a lumen (288), which are substantially similar to distal end (152), array of apertures (154), distal tip (156), and lumen (158) described above, with differences elaborated below. In particular, cannula (280) includes a plurality of barbs (285) extending from a cannula body located at distal end (282). Barbs (285) extend from cannula (280) in a proximal direction in order to engage cannula housing (130). Barbs (285) are configured to sufficiently engage cannula housing (130) in order to resist proximal movement of cannula (280) relative to wound closure device (120) after being suitably inserted within cannula housing (130). The proximal angled nature of barbs (285) may help promote initial distal insertion of cannula (280), while inhibiting proximal retraction. Barbs (285) are atraumatic such that barbs suitably engage cannula housing (130) without damaging and/or disturbing skin (S) and/or wound (W) during suitable use in accordance with the description herein.

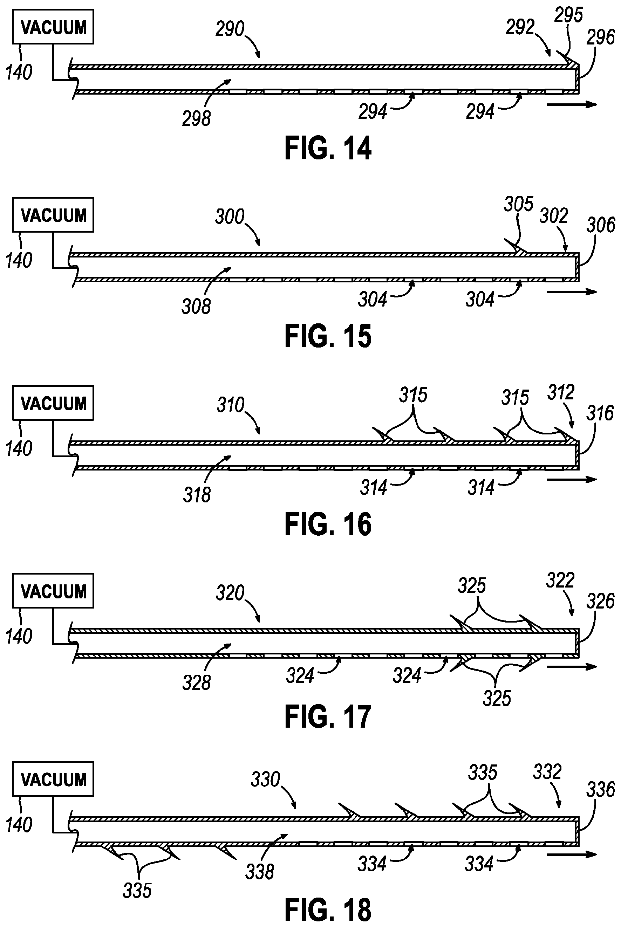

FIG. 14 shows another illustrative cannula (290) that is substantially similar to cannula (150) described above, but with differences elaborated below. Cannula (290) includes a distal end (292), an array of apertures (294), a distal tip (296), and a lumen (298), which are substantially similar to distal end (152), array of apertures (154), distal tip (156), and lumen (158) described above, with differences elaborated below. Additionally, cannula (290) includes a single barb (295) that is substantially similar to a single barb (285) of the plurality of barbs (285) described above. Single barb (295) is located directly adjacent to distal tip (296).

FIG. 15 shows another illustrative cannula (300) that is substantially similar to cannula (150) described above, but with differences elaborated below. Cannula (300) includes a distal end (302), an array of apertures (304), a distal tip (306), and a lumen (308), which are substantially similar to distal end (152), array of apertures (154), distal tip (156), and lumen (158) described above, with differences elaborated below. Additionally, cannula (300) includes a single barb (305) that is substantially similar to a single barb (295) described above. In this example, single barb (305) is located more proximally to distal tip (306) compared to single barb (295) described above.

FIG. 16 shows another illustrative cannula (310) that is substantially similar to cannula (150) described above, but with differences elaborated below. Cannula (310) includes a distal end (312), an array of apertures (314), a distal tip (316), and a lumen (318), which are substantially similar to distal end (152), array of apertures (154), distal tip (156), and lumen (158) described above, with differences elaborated below. Cannula (300) also includes a plurality of barbs (315) that are substantially similar to barbs (285) described above, except barbs (315) are spaced further apart from one another.. In this example, single barb (305) is located more proximally to distal tip (306) compared to single barb (295) described above.

FIG. 17 shows another illustrative cannula (320) that is substantially similar to cannula (150) described above, but with differences elaborated below. Cannula (320) includes a distal end (322), an array of apertures (324), a distal tip (326), and a lumen (328), which are substantially similar to distal end (152), array of apertures (154), distal tip (156), and lumen (158) described above, with differences elaborated below. Cannula (320) also includes a plurality of barbs (325) that are substantially similar to barbs (285) described above, except barbs (325) in the current example are arranged in pairs that extend in opposite directions. Therefore, two barbs (325) extend away from the direction of apertures (334), while two other barbs (325) extend in the same direction which apertures (334) are presented.

FIG. 18 shows another illustrative cannula (330) that is substantially similar to cannula (150) described above, but with differences elaborated below. Cannula (330) includes a distal end (332), an array of apertures (334), a distal tip (336), and a lumen (338), which are substantially similar to distal end (152), array of apertures (154), distal tip (156), and lumen (158) described above, with differences elaborated below. Cannula (330) also includes a plurality of barbs (325) that are substantially similar to barbs (325) described above with differences elaborated below. However, rather that being arranged in pairs, barbs (335) are arranged in a proximal array and a distal array of barbs (335). The distal array of barbs (335), two barbs (335) extend away from the direction of apertures (334), while proximal array barbs (325) extend in the same direction which apertures (334) are presented.

Versions of the devices described above may have application in conventional medical treatments and procedures conducted by a medical professional, as well as application in robotic-assisted medical treatments and procedures. By way of example only, various teachings herein may be readily incorporated into a robotic surgical system such as the DAVINCI^{™} system by Intuitive Surgical, Inc., of Sunnyvale, California.

Versions of the devices described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. An apparatus, comprising:
(a) a cannula defining a lumen, wherein the cannula is configured to operatively couple with a suction source to thereby establish fluid communication between the suction source and the lumen, wherein the cannula further comprises a distal portion defining at least one aperture, wherein the at least one aperture is in fluid communication with the lumen and an exterior surface of the distal portion; and
(b) a wound closure device dimensioned to cover a wound in skin, wherein the wound closure device is configured to receive a layer of topical adhesive to thereby adhere to the skin and form a microbial barrier over the wound, wherein the wound closure device comprises a cannula housing defining a channel dimensioned to receive the distal portion of the cannula, wherein the cannula is configured to be inserted within the cannula housing to thereby provide fluid communication between the suction source and the wound after formation of the microbial barrier;
wherein the wound closure device further comprises a textile mesh, wherein the textile mesh comprises the cannula housing.

2. The apparatus of claim 1, wherein the wound closure device further comprises a pressure sensitive adhesive configured to temporarily attach the textile mesh to the wound.

3. The apparatus of claim 2, wherein the wound closure device further comprises a backing removably covering the pressure sensitive adhesive.

4. The apparatus of any preceding claim, wherein the distal portion of the cannula further comprises a closed distal tip.

5. The apparatus of any preceding claim, wherein the distal portion of the cannula further comprises at least one barb.

6. The apparatus of claim 5, wherein the at least one barb comprises a longitudinal array of barbs.

7. The apparatus of claim 6, wherein the longitudinal array of barbs extends away from the at least one aperture of the distal portion of the cannula.

8. The apparatus of any preceding claim, wherein the wound closure device comprises a first textile mesh and a second textile mesh, wherein the distal portion of the cannula is interposed between the first textile mesh and the second textile mesh.

9. The apparatus of claim 8, wherein the second textile mesh comprises a pressure sensitive adhesive, wherein the cannula is attached to the pressure sensitive adhesive.

10. The apparatus of any preceding claim, wherein the wound closure device comprises a substantially rectangular shape.

11. The apparatus of any preceding claim, wherein the cannula housing comprises an open proximal end and a closed distal end.

12. The apparatus of claim 11, wherein the open proximal end is located at a first peripheral location of the wound closure device.

13. The apparatus of claim 11 or 12, wherein the distal end is located at a second peripheral location of the wound closure device.

14. The apparatus of any preceding claim, wherein the cannula housing comprises a proximal end and distal end, wherein the cannula housing is tapered such that the proximal end is wider than the distal end.

15. The apparatus of any preceding claim, wherein the proximal end and the distal end are both located within the peripheral edges of the wound closure device.

## Patentansprüche

1. Gerät, umfassend:
(a) eine Kanüle, die ein Lumen definiert, wobei die Kanüle konfiguriert ist, um operativ mit einer Saugquelle zu koppeln, um dadurch eine Fluidverbindung zwischen der Saugquelle und dem Lumen herzustellen, wobei die Kanüle ferner einen distalen Abschnitt umfasst, der mindestens eine Öffnung definiert, wobei die mindestens eine Öffnung in Fluidverbindung mit dem Lumen und einer Außenoberfläche des distalen Abschnitts steht; und
(b) eine Wundverschlussvorrichtung, die dimensioniert ist, um eine Wunde in der Haut abzudecken, wobei die Wundverschlussvorrichtung konfiguriert ist, um eine Schicht eines topischen Klebstoffs zu empfangen, um dadurch an der Haut zu haften und eine mikrobielle Barriere über der Wunde zu bilden, wobei die Wundverschlussvorrichtung ein Kanülengehäuse umfasst, das einen Kanal definiert, der dimensioniert ist, um den distalen Abschnitt der Kanüle aufzunehmen, wobei die Kanüle konfiguriert ist, um in das Kanülengehäuse eingeführt zu werden, um dadurch eine Fluidverbindung zwischen der Saugquelle und der Wunde nach Bildung der mikrobiellen Barriere bereitzustellen;
wobei die Wundverschlussvorrichtung ferner ein Textilnetz umfasst, wobei das Textilnetz das Kanülengehäuse umfasst.

2. Gerät nach Anspruch 1, wobei die Wundverschlussvorrichtung ferner einen druckempfindlichen Klebstoff umfasst, der konfiguriert ist, um das Textilnetz vorübergehend an der Wunde zu befestigen.

3. Gerät nach Anspruch 2, wobei die Wundverschlussvorrichtung ferner eine Verstärkung umfasst, die den druckempfindlichen Klebstoff abnehmbar abdeckt.

4. Gerät nach einem der vorstehenden Ansprüche, wobei der distale Abschnitt der Kanüle ferner eine geschlossene distale Spitze umfasst.

5. Gerät nach einem der vorstehenden Ansprüche, wobei der distale Abschnitt der Kanüle ferner mindestens einen Widerhaken umfasst.

6. Gerät nach Anspruch 5, wobei der mindestens eine Widerhaken ein Array von Widerhaken in Längsrichtung umfasst.

7. Gerät nach Anspruch 6, wobei sich das longitudinale Array von Widerhaken von der mindestens einen Öffnung des distalen Abschnitts der Kanüle weg erstreckt.

8. Gerät nach einem der vorstehenden Ansprüche, wobei die Wundverschlussvorrichtung ein erstes Textilnetz und ein zweites Textilnetz umfasst, wobei der distale Abschnitt der Kanüle zwischen dem ersten Textilnetz und dem zweiten Textilnetz angeordnet ist.

9. Gerät nach Anspruch 8, wobei das zweite Textilnetz einen druckempfindlichen Klebstoff umfasst und die Kanüle an dem druckempfindlichen Klebstoff befestigt ist.

10. Gerät nach einem der vorstehenden Ansprüche, wobei die Wundverschlussvorrichtung eine im Wesentlichen rechteckige Form umfasst.

11. Gerät nach einem der vorstehenden Ansprüche, wobei das Kanülengehäuse ein offenes proximales Ende und ein geschlossenes distales Ende umfasst.

12. Gerät nach Anspruch 11, wobei sich das offene proximale Ende an einer ersten peripheren Stelle der Wundverschlussvorrichtung befindet.

13. Gerät nach Anspruch 11 oder 12, wobei sich das distale Ende an einer zweiten peripheren Stelle der Wundverschlussvorrichtung befindet.

14. Gerät nach einem der vorstehenden Ansprüche, wobei das Kanülengehäuse ein proximales Ende und ein distales Ende umfasst, wobei das Kanülengehäuse verjüngt ist, sodass das proximale Ende breiter ist als das distale Ende.

15. Gerät nach einem der vorstehenden Ansprüche, wobei das proximale Ende und das distale Ende beide innerhalb der Umfangskanten der Wundverschlussvorrichtung angeordnet sind.

## Revendications

1. Appareil, comprenant :
(a) une canule définissant une lumière, dans lequel la canule est conçue pour s'accoupler de manière fonctionnelle à une source d'aspiration pour établir ainsi une communication fluidique entre la source d'aspiration et la lumière, dans lequel la canule comprend en outre une partie distale définissant au moins une ouverture, dans lequel l'au moins une ouverture est en communication fluidique avec la lumière et une surface extérieure de la partie distale ; et
(b) un dispositif de fermeture de plaie dimensionné pour recouvrir une plaie dans la peau, dans lequel le dispositif de fermeture de plaie est conçu pour recevoir une couche d'adhésif topique pour adhérer ainsi à la peau et former une barrière microbienne sur la plaie, dans lequel le dispositif de fermeture de plaie comprend un logement de canule définissant un canal dimensionné pour recevoir la partie distale de la canule, dans lequel la canule est conçue pour être insérée à l'intérieur du logement de canule pour fournir ainsi une communication fluidique entre la source d'aspiration et la plaie après la formation de la barrière microbienne ;
dans lequel le dispositif de fermeture de plaie comprend en outre une maille textile, dans lequel la maille textile comprend le logement de canule.

2. Appareil selon la revendication 1, dans lequel le dispositif de fermeture de plaie comprend en outre un adhésif sensible à la pression conçu pour fixer temporairement la maille textile à la plaie.

3. Appareil selon la revendication 2, dans lequel le dispositif de fermeture de plaie comprend en outre un support recouvrant de manière amovible l'adhésif sensible à la pression.

4. Appareil selon l'une quelconque revendication précédente, dans lequel la partie distale de la canule comprend en outre un embout distal fermé.

5. Appareil selon l'une quelconque revendication précédente, dans lequel la partie distale de la canule comprend en outre au moins un ardillon.

6. Appareil selon la revendication 5, dans lequel l'au moins un ardillon comprend un réseau longitudinal d'ardillons.

7. Appareil selon la revendication 6, dans lequel le réseau longitudinal d'ardillons s'étend en s'éloignant de l'au moins une ouverture de la partie distale de la canule.

8. Appareil selon l'une quelconque revendication précédente, dans lequel le dispositif de fermeture de plaie comprend une première maille textile et une seconde maille textile, dans lequel la partie distale de la canule est interposée entre la première maille textile et la seconde maille textile.

9. Appareil selon la revendication 8, dans lequel la seconde maille textile comprend un adhésif sensible à la pression, dans lequel la canule est fixée à l'adhésif sensible à la pression.

10. Appareil selon l'une quelconque revendication précédente, dans lequel le dispositif de fermeture de plaie comprend une forme sensiblement rectangulaire.

11. Appareil selon l'une quelconque revendication précédente, dans lequel le logement de canule comprend une extrémité proximale ouverte et une extrémité distale fermée.

12. Appareil selon la revendication 11, dans lequel l'extrémité proximale ouverte est située au niveau d'un premier emplacement périphérique du dispositif de fermeture de plaie.

13. Appareil selon la revendication 11 ou 12, dans lequel l'extrémité distale est située au niveau d'un second emplacement périphérique du dispositif de fermeture de plaie.

14. Appareil selon l'une quelconque revendication précédente, dans lequel le logement de canule comprend une extrémité proximale et une extrémité distale, dans lequel le logement de canule est effilé de telle sorte que l'extrémité proximale est plus large que l'extrémité distale.

15. Appareil selon l'une quelconque revendication précédente, dans lequel l'extrémité proximale et l'extrémité distale sont toutes deux situées à l'intérieur des bords périphériques du dispositif de fermeture de plaie.
